(19) Europäisches Patentamt / European Patent Office / Office européen des brevets

(11) **EP 1 902 708 A1**

(12) **EUROPÄISCHE PATENTANMELDUNG**

(43) Veröffentlichungstag:
**26.03.2008 Patentblatt 2008/13**

(21) Anmeldenummer: **06020008.6**

(22) Anmeldetag: **25.09.2006**

(51) Int Cl.:
*A61K 9/20* (2006.01)     *A61K 9/28* (2006.01)
*A61K 9/48* (2006.01)     *A61K 9/00* (2006.01)

(84) Benannte Vertragsstaaten:
**AT BE BG CH CY CZ DE DK EE ES FI FR GB GR HU IE IS IT LI LT LU LV MC NL PL PT RO SE SI SK TR**
Benannte Erstreckungsstaaten:
**AL BA HR MK YU**

(71) Anmelder: **Losan Pharma GmbH**
**79395 Neuenburg (DE)**

(72) Erfinder:
• **Gruber, Peter**
**79249 Merzhausen (DE)**
• **Spickermann, Dirk**
**79219 Staufen (DE)**

(74) Vertreter: **Kalhammer, Georg et al**
**Lederer & Keller**
**Patentanwälte**
**Prinzregentenstrasse 16**
**80538 München (DE)**

(54) **Wirkstoff enthaltende stabilisierte feste Arzneimittelformen und Verfahren zu ihrer Herstellung**

(57) Die Erfindung betrifft feste Arzneiformen enthaltend wenigstens einen Wirkstoff und wenigstens ein pharmazeutisch verträgliches, wasserlösliches Trockenmittel, das ausgewählt ist aus der Gruppe bestehend aus Trimagnesiumdicitrat und/oder Calciumchlorid, wobei die feste Arzneiform einen Trocknungsverlust von höchstens 6% und eine relative Gleichgewichtsfeuchte von 25% oder weniger aufweist. Die Erfindung betrifft auch feste Arzneiformen enthaltend einen feuchtempfindlichen Wirkstoff und Trimagnesiumdicitrat.

EP 1 902 708 A1

**Beschreibung**

Die Stabilität von festen Arzneiformen oder festen Food-Supplementformen muss während der Gesamtlaufzeit gesichert sein. Der Begriff Stabilität umfasst die chemische Stabilität des Wirkstoffes aber auch physikalisch-technologische Eigenschaften wie Fließfähigkeit, Agglomeratfreiheit, Teilbarkeit der Tabletten und sehr wichtige Aspekte wie das Aussehen, Vermeidung von Verfärbung des Produktes und insbesondere den Geschmack, wie er z. B. bei Lutschtabletten, Kautabletten, oder Formen wie Brausetabletten oder Trinkgranulate, die vor der Anwendung aufzulösen sind, sehr wichtig ist. Im pharmazeutischen Bereich ist die chemische Stabilität über die Gesamtlaufzeit des Präparates extrem wichtig, da sich der Gehalt des Wirkstoffes möglichst nicht verringern soll und auch die Bildung von Zersetzungsprodukten, die durchaus toxisch sein können, so gut wie möglich zu vermeiden ist.

Neben Sauerstoff ist insbesondere Wasser, das jeder Wirkstoff und jeder Hilfsstoff in unterschiedlichen Mengen enthält, für alle Arten der Stabilität wie z. B. chemische Stabilität, Stabilität gegenüber Verfärbung, Stabilität des Aromas verantwortlich.

Dem Fachmann ist eine Vielzahl von Möglichkeiten bekannt, wie er die Stabilität in dem breiten genannten Rahmen sichern oder zumindest verbessern kann. So ist es durchaus üblich, dass gewisse, hochempfindliche Arzneistoffe vor der Anwendung in der Tiefkühltruhe z.B. -15 bis -25°C oder mindestens im Kühlschrank zu lagern sind. Diese Maßnahme schränkt die Anwendbarkeit extrem ein und erhöht die Kosten für das Arzneimittel deutlich.

Weit verbreitet ist die Maßnahme, die Wirkstoffe und gegebenenfalls auch alle mit den Wirkstoffen verbundenen Hilfsstoffe vor Herstellung der festen Formen zu trocknen. Dabei treten jedoch z. T. enorme Probleme auf. Getrocknete Pulver oder Granulate z.B. zeigen extreme elektrostatische Aufladungen und verlieren dadurch ihre Fließfähigkeit und lassen sich dadurch auf Produktionsmaschinen z.B. nicht in Kapseln oder Sachets abfüllen. Die bei der Verpressung von Tabletten entstehende Bindung ist ohne die Mitwirkung von Wasser nicht möglich. Deshalb können aus zu stark getrockneten Granulaten keine mechanisch ausreichend festen Tabletten hergestellt werden. Jeder thermische Trocknungsvorgang birgt die Gefahr einer bereits einsetzenden Zersetzung des empfindlichen Wirkstoffes.

Schließlich besteht für den Fachmann noch die Möglichkeit, die genannten festen Formen nach der Herstellung zu trocknen. Aber auch hier entstehen Probleme, da z.B. getrocknete Kapseln und Tabletten elektrostatisch aufgeladen sein können, und nicht mehr auf schnell laufenden Verpackungsmaschinen störungsfrei in die Behältnisse gefüllt werden können. Hartgelatine-Kapseln z.B. verlieren ihre Flexibilität und sind ebenfalls nach der Trocknung elektrostatisch aufgeladen und nicht unter den Gesichtspunkten einer wirtschaftlichen Pharmaproduktion abfüllbar.

Aber auch hier bietet sich dem Fachmann letztlich noch die Möglichkeit, die festen Formen in Behältnisse wie dicht schließende Glasfläschchen, Polyethylen (PE)-Dosen oder Aluminium-Röhrchen abzufüllen und mit einem Trockenstopfen zu versehen, der ein geeignetes Trockenmittel wie Silicagel oder Molekularsieb enthält. Diese letzte Möglichkeit zur Stabilisierung feuchtempfindlicher fester Formen hat jedoch ebenfalls zahlreiche Nachteile. Solche Flaschen werden in der Regel von Patienten abgelehnt, da sie in der Handhabung wesentlich unpraktischer sind als Blister-Streifen. Der Trockenstopfen selbst stellt eine teure Packmittelkomponente dar und das in dem Stopfen befindliche Trockenmittel hat nur eine beschränkte Aufnahmekapazität. Werden z.B. 50 oder 100 Tabletten mit einem Einzelgewicht von 500 mg abgefüllt, ist der Trockenstopfen nicht in der Lage, die zur Stabilisierung des Produktes notwendige Wassermenge aus den Tabletten herauszuziehen und zu adsorbieren. Der größte Nachteil jedoch insbesondere bei sehr empfindlichen Wirkstoffen besteht darin, dass die Austrocknung der festen Form in dem dichten Packmittel in der Regel Wochen dauert und in dieser Zeit der feuchtempfindliche Wirkstoff bereits zu stark zersetzt ist. Außerdem wie im weiteren Verlauf der Erfindung noch gezeigt wird, besteht ein extremes Gefälle der Austrocknung der festen Formen in Abhängigkeit von der Entfernung der festen Form von dem Trockenstopfen. So sind in der Regel Tabletten oder Kapseln in der Nähe des Trockenstopfens übertrocknet und weiter entfernte feste Formen durch einen nicht ausreichenden Wasserentzug ungenügend stabilisiert. Dieses Problem ist auch nicht lösbar, wenn anstelle eines Trockenstopfens eine Trockenkapsel in das Produkt selbst zwischen die abgefüllten Tabletten z.B. in einer Flasche eingelegt wird.

Eine letzte Möglichkeit - insbesondere für Wirkstoffe, die gegenüber Feuchtigkeit hoch empfindlich sind - würde darin bestehen, die genannten festen Formen zu trocknen und gegebenenfalls (aufgrund elektrostatischer Aufladungen) per Hand in Räumen mit einer relativen Feuchtigkeit < 10%, bevorzugt < 5%, in dichte Packmittel abzufüllen, damit das durch die Trocknung hochhygroskopische Produkt keine Gelegenheit hat, größere Mengen an Feuchtigkeit, welche die Stabilität des Produktes gefährden, aufzunehmen. Eine solche Produktion ist nur in Einzelfällen, z.B. bei der Herstellung von gefriergetrockneten Produkten, die sehr teuer sind, durchführbar. Die Sicherstellung der genannten klimatischen Bedingungen in Produktionsräumen ist sehr teuer und die Belastung der Mitarbeiter selbst unter diesen extrem trockenen Bedingungen über Stunden nicht akzeptabel.

Die genannten Probleme können in Einzelfällen dadurch überwunden werden, dass die feste Form mit dem Trockenmittel Siliciumdioxid (z.B. Syloid AL 1, Handelsname) gemischt wird. Syloid ist allgemein als Trockenmittel bekannt und ist in der Lage, unter der Bedingung 25°C / 10% relative Luftfeuchtigkeit einige Prozent Wasser fest zu binden. Dies bedeutet mit anderen Worten, dass das eingemischte Siliciumdioxid z.B. aus den Granulat- oder aus den Tablettenbestandteilen (einschließlich des Wirkstoffs), Wasser entzieht und direkt selbst aufnimmt.

Siliciumdioxid hat jedoch den großen Nachteil, dass es ein extrem feines, staubiges Produkt ist, in der Regel mit einer Partikelgröße von wenigen Mikrometer, so dass es bekanntermaßen auch als Fließreguliermittel eingesetzt wird. Es ist in Wasser unlöslich und insbesondere bei einem Zusatz von mehreren Prozent zu der festen Form ist die Mischung nicht verpressbar. Die Bindungsfestigkeit des Wassers ist bereits bei 40°C stark reduziert, so dass bei Anwendung der weltweit geltenden Richtlinien für die Stressstabilitätsuntersuchungen im pharmazeutischen Bereich (40°C, 75% relative Feuchtigkeit) nur enttäuschende Ergebnisse resultieren.

Klassische Trockenmittel wie Phosphorpentoxid, Calciumoxid, Calciumsulfat, Silicagel werden aus verschiedenen Gründen ausgeschlossen. Phosphorpentoxid und Calciumoxid sind viel zu aggressiv und führen mit zahlreichen Wirk- und Hilfsstoffen zu Inkompatibilitäten. Calciumsulfat und Silicagel sind unlöslich und enttäuschen insbesondere bezüglich der Stärke der Wasserbindung bei 40°C. Molekularsieb wäre ein bedingt geeignetes internes Trockenmittel, ist jedoch für orale Zwecke nicht erlaubt und würde auch keine ausreichenden Kompressionseigenschaften aufweisen und ist unlöslich.

Eine Aufgabe der vorliegenden Erfindung ist es daher, eine feste Arzneiform bereitzustellen, die lagerstabil und für Wirkstoffe geeignet ist, die sehr empfindlich gegen Feuchtigkeit sind.

Diese Aufgabe wird durch den Gegenstand der Patentansprüche gelöst.

Überraschenderweise wurde gefunden, dass durch Verwendung von bestimmten wasserlöslichen Trockenmitteln, die nicht nur eine hohe Trocknungskapazität aufweisen, sondern auch das Wasser fest binden können, feste Arzneiformen mit ausgezeichneter Lagerstabilität hergestellt werden können. Insbesondere feuchtempfindliche Wirkstoffe zeigen in den erfindungsgemäßen Arzneiformen eine sehr gute Stabilität.

Völlig überraschend für den Fachmann sind beispielsweise die Ergebnisse des Versuchs mit Amoxicillin-Trihydrat (siehe Beispiel 2), der belegt, dass getrocknetes Trimagnesiumdicitrat eine höhere Bindungstendenz zum Wasser hat als das bekannte Trocknungsmittel Silicagel. Im Gegensatz zu Silicagel ist getrocknetes Trimagnesiumdicitrat in der Lage, das gesamte Kristallwasser von Amoxicillin-Trihydrat zu binden. Durch diesen Versuch ist in besonderer Weise bewiesen, dass in eine feste Form eingemischtes getrocknetes Trimagnesiumdicitrat in der Lage ist, größere Mengen an Wasser zu binden und die Bestandteile dieser Mischung, insbesondere einen feuchtempfindlichen Wirkstoff so zu trocknen, dass eine durch Wasser verursachte Zersetzung des Wirkstoffes weitgehend vermieden wird. Auch mit Calciumchlorid konnten sehr vorteilhafte Ergebnisse erzielt werden.

Die Erfindung betrifft daher eine feste Zusammensetzung, vorzugsweise eine feste Arzneiform, enthaltend wenigstens einen Wirkstoff und wenigstens ein pharmazeutisch verträgliches, wasserlösliches Trockenmittel, das ausgewählt ist aus Trimagnesiumdicitrat, Calciumchlorid und Kombinationen davon, dadurch gekennzeichnet, dass die feste Zusammensetzung einen Trocknungsverlust, gemessen bei 120°C/30 min, von höchstens 6% und eine relative Gleichgewichtsfeuchte, gemessen bei 25°C, von 25% oder weniger aufweist.

Die bevorzugten Trocknungsmittel gemäß der vorliegenden Erfindung weisen folgende Eigenschaften auf:

Sie sind

- physiologisch unbedenklich, kostengünstig und weltweit im pharmazeutischen und Lebensmittelbereich akzeptiert;
- wasserlöslich, in den eingesetzten Mengen nicht schlecht schmeckend;
- verfügbar und produktionstechnisch gut verarbeitbar in einem mittleren Korngrößenbereich von 0,02 bis 2,5 mm;
- problemlos verpressbar, kompaktierbar.
- chemisch kompatibel mit einer Vielzahl von Wirkstoffen, insbesondere mit feuchtempfindlichen Wirkstoffen wie z.B. Acetylsalicylsäure (ASS), Clavulansäure, Protonenpumpeninhibitoren wie Omeprazol, Lansoprazol, Pantoprazol und zahlreichen feuchtempfindlichen Prodrugs.

Sie haben

- praktisch keinen negativen Einfluss auf die Freisetzungsgeschwindigkeit von Wirkstoffen aus den genannten festen Formen;
- möglichst hohe und feste Adsorptionsfähigkeit von Wasser in dem Bereich bis zu 25% relativer Feuchte und einer Temperatur von maximal 40°C;
- als 0,1 molare Lösung einen pH-Wert von 5 bis 8, bevorzugt von 5,5 bis 7,5;

Geeignete Trockenmittel sind Calciumchlorid, Magnesiumsulfat, Tricalciumcitrat, Carnitin. Zur Überraschung des Fachmannes erfüllt jedoch Trimagnesiumdicitrat am besten die oben genannten bevorzugten Eigenschaften eines internen Trockenmittels. Es handelt sich um ein preiswertes, physiologisch völlig unbedenkliches, wasserlösliches Salz mit ausreichend guten Kompressions- und Kompaktiereigenschaften. Es ist sowohl das 12-Hydrat als auch das 9-Hydrat und eine getrocknete Form bekannt. Das Salz ist sowohl in mikronisierter Form mit einer mittleren Korngröße von 25 µm wie auch in einer mittleren Korngröße von 1 bis 2 mm erhältlich.

Die erfindungsgemäße Arzneiform weist einen Trocknungsverlust von höchstens 6%, vorzugsweise von höchstens 4,5%, bevorzugter von höchstens 3,5%, noch bevorzugter von höchstens 3,0%, am bevorzugtesten von 2,5% oder weniger auf, zum Beispiel 0,1% bis 2,5% oder 0,5% bis 2,5%.

Der Trocknungsverlust ist ein Maß für die Menge an Wasser in einer festen Form:

$$\text{Trocknungsverlust in \%} = \frac{\text{Masse des Wassers in der Probe}}{\text{Gesamtmasse der Probe}} \times 100$$

Zur Bestimmung des Trocknungsverlusts einer Probe (zum Beispiel einer festen Arzneiform) wird auf einer so genannten Trocknungswaage die Probe ausgebreitet und (z.B. durch einen exakt arbeitenden Infrarotstrahler) auf die gewünschte Temperatur z.B. von 105°C eingestellt. Nach einer bestimmten Zeit (z.B. nach 20 oder 30 Minuten) wird der Trocknungsverlust bedingt durch das Verdampfen von Wasser in Prozent direkt an der Waage abgelesen. Sofern in dieser Anmeldung nichts anderes angegeben ist, bezieht sich der Ausdruck "Trockungsverlust" auf den Trockungsverlust, der unter den Bedingungen 105°C / 30 min ermittelt wurde. Ein geeignetes Gerät zur Messung des Trocknungsverlustes ist der Moisture Analyzer HR83 der Firma Mettler-Toledo (CH-8606 Greifensee, Schweiz). Vorzugsweise wird das Gerät bzw. die Trocknungswaage in einem geschlossenen Innenraum mit einer Umgebungstemperatur von 20°C und 20% relativer Feuchte eingesetzt.

Der Trocknungsverlust sagt nichts über die Festigkeit der Bindung des Wassers aus und erlaubt keine Aussage, wie stark und in welcher Form das Wasser gebunden ist. Theoretisch gibt es Haftwasser, Kapillarwasser, Quellungswasser, absorbiertes Wasser und Kristallwasser. Ersteres ist ungebunden und verdampft sehr leicht, das Kristallwasser wiederum kann sehr fest gebunden sein und erfordert bei der Bestimmung des Trocknungsverlustes eine hohe Temperatur und eine lange Trocknungszeit.

Im Zusammenhang mit der vorliegenden Erfindung ist es jedoch sehr wichtig, über eine Methode zu verfügen, die etwas über den Bindungsmechanismus des Wassers und die Stärke der Wasserbindung aussagt. Dazu wird die so genannte "relative Gleichgewichtsfeuchte" (im folgenden auch kurz als "Gleichgewichtsfeuchte" bezeichnet) gemessen, die sich in einer vollkommen dichten Messzelle bei einer bestimmten Temperatur über der Probe bildet. Sie wird auch als Gleichgewichtsaktivität oder Wasseraktivität bezeichnet. Die Wasseraktivität beschreibt die Bindungsstärke des Wassers an den Wirkstoff bzw. an die einzelnen Komponenten der Wirkstoffmischung. Der gemessene Wert für die Wasseraktivität bzw. für die Gleichgewichtsfeuchte (Equilibrium Relative Humidity) hat einen entscheidenden Einfluss auf die chemische Stabilität eines feuchtempfindlichen Wirkstoffes, der in einer festen Form wie z.B. ein Granulat, eine Kapsel oder Tablette eingebettet ist.

Die Gleichgewichtsfeuchte ist der Wert der relativen Feuchte, in welcher ein hygroskopisches Produkt gelagert werden kann, ohne dass ein Netto-Austausch von Feuchtigkeit zwischen dem Produkt und seiner Umgebung stattfindet. Hat also eine feste Form eine Gleichgewichtsfeuchte von 10% und wird bei 15% relativer Feuchte gelagert, so nimmt es Wasser auf seiner Umgebung auf, ist die relative Feuchte der Umgebung unter 10% so gibt die feste Form Wasser an seine Umgebung ab und trocknet noch stärker aus.

Die Korrelation zwischen der Gleichgewichtsfeuchte und dem Feuchtigkeitsgehalt einer festen Form (Trocknungsverlust) kann über die bekannte Sorptionsisotherme dargestellt werden, wobei auf der Abszisse die Gleichgewichtsfeuchte bzw. die Wasseraktivität angegeben ist und auf der Ordinate in Prozent der Wassergehalt.

Sofern hierin nichts anderes angegeben ist, wird die Gleichgewichtsfeuchte in einer thermostatisierten kleinen, ca. 50 ml Volumen großen, dicht schließenden Messzelle wie folgt bestimmt. Im oberen Teil dieser Messzelle befindet sich ein Sensor, der die durch die Verdunstung des Wassers aus der zu messenden Probe entstehende relative Feuchtigkeit messen kann (zur Anwendung kommt das Gerät HygroLab mit der Messzelle AWVC der Firma Rotronic, CH 8303 Bassersdorf, Schweiz). Das Gerät ist amtlich kalibriert und die Messunsicherheit beträgt nicht mehr als ± 1% relative Feuchte, bzw. ± 0,3°C. Die Messgenauigkeit kann jederzeit mit standardisierten Salzlösungen, wie sie dem Fachmann bekannt sind, wiederholt werden. Die Messung findet bei 25°C statt.

Misst man z.B. kristalline Zitronensäure mit einem Trocknungsverlust von 0,2% (gemessen bei 70°C/30 min), so ergibt die Messung der auf 25°C thermostatisierten Messzelle eine Gleichgewichtsfeuchte von 63%. Misst man dagegen Maisstärke mit 8,5% Trocknungsverlust (bestimmt ebenfalls bei 70°C/Messzeit 30 min), so kann man eine Gleichgewichtsfeuchte von nur 22% messen. Der Vergleich der beiden Messwerte zeigt klar, dass das Wasser bei der Zitronensäure im Gegensatz zur Maisstärke nur sehr locker gebunden ist und damit z.B. für die Zersetzung eines feuchtempfindlichen Wirkstoffes leicht zur Verfügung steht.

Die erfindungsgemäßen Trockenmittel, die nicht nur stark hygroskopisch sind, sondern auch das aufgenommene Wasser fest binden, können in Kombination mit feuchtempfindlichen Wirkstoffen eine durch Wasser hervorgerufene Zersetzung oder eine Verfärbung usw. fast vollständig unterdrücken.

Durch die Art und Menge des verwendeten Trockenmittels, welches zusammen mit dem feuchtempfindlichen Wirkstoff in die feste Form eingearbeitet ist, kann die Gleichgewichtsfeuchte in der festen Form so erniedrigt werden, dass fast kein freies Wasser zur Zersetzung des Wirkstoffes zur Verfügung steht.

Die erfindungsgemäße Arzneiform weist eine Gleichgewichtsfeuchte, gemessen bei 25°C, von höchstens 25%, vorzugsweise von höchstens 20%, bevorzugter von höchstens 15%, noch bevorzugter von höchstens 10%, am bevorzugtesten von höchstens 6% auf, zum Beispiel 1% bis 6 %, oder 1,5% bis 4%, oder sogar 1,5% bis 2,5%.

Die Konzentration des Trockenmittels in der Arzneiform ist 10 bis 99,0%, vorzugsweise 15 bis 75%, bevorzugter 20 bis 50%, am bevorzugtesten 30 bis 40%. Sofern nichts anderes angegeben ist sind %-Angaben in dieser Anmeldung, die sich auf die Konzentration von Stoffen in der festen Arzneiform beziehen, Gewichts-%.

Die Konzentration des Wirkstoffs in der Arzneiform ist in der Regel von 1 % bis 75%, bevorzugter von 5% bis 50%, noch bevorzugter 10% bis 45%, am bevorzugtesten 20% bis 40%, zum Beispiel 30% bis 40%.

Der Wirkstoff kann jede Substanz sein, die eine therapeutische und/oder vorbeugende Wirkung hervorrufen kann. Vorzugsweise ist der Wirkstoff eine organische Verbindung, also ein Molekül, das Kohlenstoff enthält. Besonders geeignet sind die Arzneiformen der vorliegenden Erfindung aber für Wirkstoffe, die empfindlich gegen Feuchtigkeit sind (in der vorliegenden Anmeldung als feuchtempfindliche Wirkstoffe bezeichnet). Ein Wirkstoff gilt im Sinne der vorliegenden Anmeldung als feuchtempfindlich, wenn ein Muster des reinen Wirkstoffes, das auf eine Gleichgewichtsfeuchte von 25% bei 20°C eingestellt wurde, innerhalb von 6 Monaten bei 40°C in einem dichten Packmittel eine Zersetzung von mehr als 1% aufweist. In Abhängigkeit von der Toxizität des oder der entstehenden Zersetzungsprodukte kann die Zersetzung auch bis zu 3%, in ganz seltenen Fällen bis zu 5% betragen. 40°C ist die im pharmazeutischen Bereich weltweit abgestimmte und harmonisierte maximale Stresstemperatur für Wirkstoffe und Arzneiformen.

Beispiele für feuchtempfindliche Wirkstoffe sind ASS, Clavulansäure, Omeprazol und Lansoprazol. Der Ausdruck "Wirkstoff" im Sinne der vorliegenden Erfindung schließt so genannte Prodrugs ein, also Vorstufen der tatsächlich pharmakologisch aktiven Substanzen, die erst nach Verabreichung im Organismus in einen oder mehrere aktive Metaboliten umgewandelt werden. Gründe für die Entwicklung eines Prodrugs können schlechte Resorbierbarkeit (Enalapril-Enalaprilat), schlechte Löslichkeit, ein hoher First-Pass-Effekt oder hohe Toxizität der aktiven Substanz sein. Prodrugs sind oft feuchtempfindlich. Dazu zählen insbesondere Ester- und Amidverbindungen, die in Gegenwart von Wasser zur Hydrolyse neigen.

Die Erfindung betrifft daher weiterhin eine feste Arzneiform enthaltend 1 bis 75 Gew.-% feuchtempfindlichen Wirkstoff, 10 bis 95 Gew.-% Trimagnesiumdicitrat und gegebenenfalls weitere Hilfsstoffe. Die bevorzugten Ausführungsformen dieses Aspekts der Erfindung entsprechen den bevorzugten Ausführungsformen, die im Zusammenhang mit anderen Aspekten der Erfindung beschrieben wurden.

Der Wirkstoff ist unterschiedlich von dem Trockenmittel, das auch in der Arzneiform vorhanden ist. So enthält beispielsweise die Arzneiform, in der Trimagnesiumdicitrat als Trockenmittel enthalten ist, einen Wirkstoff, der von Trimagnesiumdicitrat und $Mg^{2+}$ verschieden ist.

Die Arzneiform der vorliegenden Erfindung ist nicht besonders eingeschränkt. Es kann sich beispielsweise um Pulver, Granulate, Tabletten, Kapseln oder andere feste Formen handeln. Bezüglich des Verwendungszwecks können die Arzneiformen für die perorale, orale, parenterale oder externe Anwendung hergerichtet sein. Es können sog. Standard-Tabletten, Minitabletten (Durchmesser <4 mm), Dragee-Kerne, Kautabletten, orodispersible Tabletten, Manteltabletten, Mehrschichttabletten, Retardtabletten nach dem Umhüllungs- oder Einbettungsprinzip, Gerüsttabletten, Matrixtabletten, Schwimmtabletten, bioadhäsive Tabletten, Filmtabletten, Magensaftresistente Tabletten, dickdarmlöslich überzogene Tabletten, Brausetabletten, sein. Diese und weitere Tablettenarten sind beschrieben in "Die Tablette" W. A. Ritschel, A. Bauer-Brandl, Editio Cantor Verlag Aulendorf, 2002, dessen Inhalt hiermit in diese Anmeldung einbezogen wird.

Die Arzneiform kann darüber hinaus weitere übliche Hilfsstoffe enthalten, wie sie in "Die Tablette" W. A. Ritschel, A. Bauer-Brandl, Editio Cantor Verlag Aulendorf, 2002 beschrieben sind, dessen Inhalt hiermit in diese Anmeldung einbezogen wird. Die Arzneiform kann mikrokristalline Cellulose, Laktose, Mannit, Xylit und Sprengmittel wie quervernetztes Povidon oder quervernetzte Natriumcarboxymethylcellulose enthalten. Als Fließreguliermittel ist z. B. Siliciumdioxid (mit dem Handelsnamen Syloid AL 1) besonders geeignet. Typische Schmiermittel für die Herstellung von Tabletten wie Magnesiumstearat und Stearinsäure, können auch enthalten sein. Vorzugsweise enthält die Arzneiform mikrokristalline Cellulose, Lactose, Mannit, Xylit, Tricalciumphosphat, Schmiermittel, Fließreguliermittel und/oder Gleitmittel. Die Konzentration an mikrokristalliner Cellulose ist vorzugsweise 5 bis 30%, bevorzugter 10 bis 20%, die des/der Schmiermittel 0,3 bis 2%, die des/der Fließreguliermittel 0,2 bis 2,0% und die des/der Gleitmittel 1,0 bis 5%. Schließlich können auch noch übliche Füllmittel enthalten sein.

Ein weiterer Aspekt der Erfindung ist eine dichte Verpackung enthaltend eine feste Arzneiform, wie sie hierin beschrieben ist. Der Ausdruck "dicht" bedeutet, dass die Verpackung im wesentlichen undurchlässig für Wasser ist. Die Verpackung kann verschiedene Formen haben. Geeignet sind z.B. die standardmäßig bekannten Packmittel wie Sachet und Stick Pack, hergestellt aus aluminiumkaschierten Folien, Glasflaschen, PE- oder PP-Röhren mit zuverlässig dicht schließenden Stopfen oder Drehverschlüssen mit Dichteinlagen. Für extrem feuchtempfindliche Wirkstoffe ist eine Aluminiumröhre mit einem festsitzenden PE-Stopfen besonders geeignet. Hervorragend haben sich auch Glasflaschen bewährt, die mit einem dem Fachmann bekannten Pilferproof-Verschluss geschlossen wurden. Auch Blisterstreifen aus Aluminiumfolie sind möglich.

Ein weiterer Aspekt der Erfindung ist eine erfindungsgemäße feste Arzneiform, verpackt in einem dichten Packmittel, wie es oben beschrieben ist.

Vorzugsweise besitzt die feste Form für wenigstens 3 Monate, vorzugsweise für wenigstens 6 Monate, bevorzugter für wenigstens 1 Jahr, am bevorzugtesten während der Gesamtlaufzeit des Produkts in dem Packmittel eine Gleichgewichtsfeuchte von höchstens 25%, vorzugsweise von höchstens 20%, bevorzugter von höchstens 15%, noch bevorzugter von höchstens 10%, am bevorzugtesten von höchstens 6%, zum Beispiel 1% bis 6%, oder 1,5% bis 4%, oder sogar 1,5% bis 2,5%, jeweils gemessen bei 25°C.

Neben Tabletten sind Kapseln bewährte Ausführungsformen dieser Erfindung. Leider sind Hartgelatine-Kapseln nur bedingt geeignet, da die Gelatine zuviel Wasser enthält. Werden die Gelatinekapseln getrocknet, so verspröden sie stark, sind elektrostatisch aufgeladen und verlieren ihre Flexibilität. Deshalb sind Hydroxypropylmethylcellulose (HPMC)-Kapseln bevorzugt, die grundsätzlich deutlich weniger Wasser enthalten. Die Zerfallszeit dieser Kapseln ist zwar etwas verlängert, sie haben jedoch den Vorteil, dass selbst stark ausgetrocknete Kapseln ihre Flexibilität bewahren.

Eine bevorzugte Ausführungsform der vorliegenden Erfindung ist somit z. B. eine HPMC-Kapsel, die in einer Glasflasche mit dicht sitzendem Stopfen abgefüllt wurde. Das Füllgut der Kapsel besteht aus 30 bis 40% hochfeuchtempfindlichen Wirkstoff, 30 bis 40% Trimagnesiumdicitrat, 10 bis 20% getrockneter mikrokristalliner Cellulose, 1 bis 2% Schmiermittel und 1 bis 3% Gleitmittel wie Talk oder bevorzugt Siliciumdioxid. Der Trocknungsverlust des Kapselinhaltes beträgt unter der scharfen Trocknungsbedingung 120°C/30 min 0,5 bis 3,5%, bevorzugt 1,5-3,0%, die Gleichgewichtsfeuchte des Inhaltes weniger als 10%, bevorzugt weniger als 6%. Der Trocknungsverlust der HPMC-Kapsel beträgt 1,5 bis 2,5%. Ganz entscheidend ist jedoch die Gleichgewichtsfeuchte, die in den genannten Grenzen auf jeden Fall einzuhalten ist.

Die Kapsel zerfällt in dem Zerfallstest in Wasser, der exakt in der Europäischen Pharmacopoe EP 5.0 beschrieben ist, zwischen 6 und 12 Minuten, in künstlichem Magensaft innerhalb von 5 bis 10 Minuten

Wird anstelle des bevorzugten Trockenmittels Trimagnesiumdicitrat Calciumchlorid eingesetzt oder Mischungen aus beiden, so verändern sich die bevorzugten Gewichtsverhältnisse wie vorstehend angegeben nicht. Der Trocknungsverlust des Kapselinhaltes liegt bei 2 bis 6 Prozent, der Trocknungsverlust der Kapsel ist unverändert, jeweils unter den gleichen Trocknungsbedingungen gemessen. Die Gleichgewichtsfeuchte des Kapselinhaltes liegt ebenfalls bevorzugt unter 6%.

Bei besonders feuchtempfindlichen Wirkstoffen oder im Falle, dass relativ große Mengen an feuchtempfindlichen Wirkstoff zu Lasten einer ausreichenden Menge an Trockenmittel verarbeitet werden müssen bzw. bei besonders langen Expositionszeiten der festen Formen während Herstellung und Abfüllung kann im Einzelfall das in einer Röhre oder Flasche abgefüllte Produkt auch mit einem Trockenmittel-Stopfen verschlossen werden. Hier bewähren sich insbesondere Trockenstopfen, die mit dem besonders aktiven Trocknungsmittel Molekularsieb gefüllt sind.

Ein weiterer Aspekt der vorliegenden Erfindung ist ein Verfahren zur Herstellung einer festen Arzneiform. In einer Ausführungsform umfasst das Verfahren die folgenden Schritte:

a) Mischen wenigstens eines Wirkstoffs mit einem Trockenmittel, das ausgewählt ist aus der Gruppe bestehend aus Trimagnesiumdicitrat, Calciumchlorid und Kombinationen davon,

b) gegebenenfalls Trockengranulierung der in Schritt a) erhaltenen Zusammensetzung, um ein Granulat zu erhalten,

c) komprimieren oder verkapseln der in Schritt a) erhaltenen Zusammensetzung oder des in Schritt b) erhaltenen Granulats, um die feste Arzneiform zu erhalten, und

d) gegebenenfalls Verpacken der festen Arzneiform in ein dichtes Packmittel.

Dabei soll das Verhältnis von Trockenmittel zu Wirkstoff in Schritt a) so gewählt werden, und das Verfahren unter solchen Bedingungen durchgeführt wird, dass die in Schritt c) erhaltene feste Arzneiform oder die in Schritt d) erhaltene feste, verpackte Arzneiform einen Trocknungsverlust, gemessen bei 120°C/30 min, von höchstens 6% und eine relative Gleichgewichtsfeuchte, gemessen bei 25°C, von 25% oder weniger aufweist.

Das bevorzugte Trockenmittel in dem Verfahren der Erfindung ist getrocknetes Trimagnesiumdicitrat. Das Trockenmittel kann vor dem Mischen mit dem Wirkstoff getrocknet werden, z.B. bei 100 bis 150°C, vorzugsweise bei 110 bis 140°C, am bevorzugtesten 120 bis 135°C. Die Trocknungszeit kann wenigstens 0.5 h, bevorzugt wenigstens 1 h, bevorzugter wenigstens 2 h, am bevorzugtesten wenigstens 3 h betragen, z.B. 1 bis 6 h, 2 bis 5 h oder 3 bis 4 h. Alternativ kann ein im wesentlichen wasserfreies Trockenmittel eingesetzt werden, das dann nicht vorgetrocknet werden muss falls der Trocknungsverlust max. 3,0% oder weniger ist (gemessen bei 150°C/30min)

Der maximale Trocknungsverlust für das Calciumchlorid darf max. 5,0% betragen (gemessen bei 150°C/30 min).

Das einzusetzende Trockenmittel sollte unmittelbar vor dem Mischen mit dem Wirkstoff einen Trocknungsverlust von weniger als 3%, vorzugsweise weniger als 2%, am bevorzugtesten weniger als 1 % aufweisen. Die Gleichgewichtsfeuchte des Trockenmittels unmittelbar vor dem Mischen mit dem Wirkstoff sollte weniger als 5%, vorzugsweise weniger als 3%, am bevorzugtesten weniger als 1 % sein.

Das Verhältnis der Masse des Trockenmittels zu der Masse des Wirkstoffs in Schritt a) kann 0,5 bis 2 sein, vorzugsweise ist es 0,75 bis 1,5, am bevorzugtesten 1 bis 1,3.

Wie dem Fachmann bekannt ist und auch eingangs bereits erwähnt wurde, ist es praktisch nicht möglich, Pulvermischungen oder Granulate mit einer zu geringen Gleichgewichtsfeuchte aufgrund von elektrostatischen Aufladungen oder mangelnder Kompressionsfähigkeit abzufüllen oder zu verpressen (siehe "Die Tablette" W. A. Ritschel, A. Bauer-Brandl, Editio Cantor Verlag Aulendorf, 2002, Seite 286). Durch geeignete Trockenmittel wie Calciumchlorid und bevorzugt getrocknetes Trimagnesiumdicitrat ist es jedoch möglich, z.B. in Pulvermischungen, Granulaten, Tabletten usw. mit einem feuchtempfindlichen Wirkstoff eine so niedrige Gleichgewichtsfeuchte zu realisieren, dass der empfindliche Wirkstoff stabilisiert wird bzw. überhaupt in feste Formen eingearbeitet werden kann.

Dieser Zusatz kann z.B. unmittelbar vor der Abfüllung eines Granulates erfolgen. Durch die Vermeidung der Trocknung zur Entfernung werden elektrostatische Aufladungen vermieden und der Abfüllvorgang in ein dichtes Packmittel wie ein Stick Pack oder ein Sachet, bestehend aus aluminiumkaschierter Folie, erfolgen. Das bevorzugte homogen verteilte Trockenmittel Trimagnesiumdicitrat ist in seiner Menge durch den Fachmann so bemessen, dass es sowohl das bei dem Abfüllvorgang absorbierte Wasser als auch das in der Gesamtmischung vorhandene Wasser so fest bindet, dass es nicht mehr zur Zersetzung des Wirkstoffes zur Verfügung steht. Wie das Beispiel 3 belegt, wird durch den Zusatz des physiologisch vollkommen unbedenklichen und geschmacklich unproblematischen Trimagnesiumdicitrates die in dem dichten Packmittel befindliche ASS-Granulatmischung so ausgetrocknet, dass die Zersetzung des Wirkstoffes ASS deutlich minimiert wird. Durch die Reduzierung der Gleichgewichtsfeuchte werden auch die Aromen stabilisiert, so dass neben der chemischen Stabilität des Wirkstoffes auch die sensorische Stabilität des Aromas während der Gesamtlaufzeit des Präparates deutlich verbessert ist.

Selbstverständlich ist es ein Vorteil, wenn bei hochempfindlichen Wirkstoffen die Herstellung der festen Form und die Abfüllung unter klimatischer Kontrolle erfolgt. Hier haben sich im pharmazeutischen Produktionsbereich Bedingungen von 20 bis 25% relativer Feuchtigkeit bei etwa 20°C (oder 15 bis 25 oder 18 bis 22°C) weltweit bewährt. Durch die reduzierte Feuchtigkeit in den Räumen wird somit die Aufnahme von Feuchtigkeit bei den Arbeitsoperationen wie Sieben, Mischen, Tablettieren und Abfüllen verringert. Wenn man ohne die erfindungsgemäßen Trockenmittel arbeitet, reichen die genannten klimatischen Bedingungen von 20% bei feuchtempfindlichen Wirkstoffen bei weitem nicht aus, ein über Jahre stabiles Produkt zu garantieren, da aus Stabilitätsgründen die Gleichgewichtsfeuchte des Produktes oft deutlich unter 10% liegen muss. Ein solches Produkt würde nur dann keine Feuchtigkeit aufnehmen, wenn in dem Produktionsraum die relative Feuchtigkeit ebenfalls unter 10% liegen würde.

Wie in Beispiel 4 gezeigt wird, kann durch erfindungsgemäßes Vorgehen der sehr feuchtempfindliche Wirkstoff Clavulansäure hervorragend stabilisiert werden. Das Beispiel belegt, dass durch die Zugabe von getrocknetem Trimagnesiumdicitrat die Gleichgewichtsfeuchte der abzufüllenden Mischung bei nur 5% (25°C) liegt. Ungeachtet dieser extrem niedrigen Gleichgewichtsfeuchte kann das Produkt trotzdem in Produktionsräumen bei 25%/20°C bedenkenlos abgefüllt werden. Die während des Abfüllvorganges unvermeidlich aufgenommene Feuchtigkeit wird durch das bevorzugte Trimagnesiumdicitrat fest gebunden und verändert zur Überraschung des Fachmannes die Gleichgewichtsfeuchte der in einer dichten Flasche abgefüllten Pulvermischung nicht. Bei einer Gleichgewichtsfeuchte von nur 5% ist die Clavulansäure nur noch in einer sehr geringen Wassermenge in Kontakt, so dass die Stabilität des Wirkstoffes durch die erfinderischen Maßnahmen deutlich verbessert werden kann. Sind durch komplizierte und länger dauernde unvermeidliche Herstellvorgänge die Expositionszeiten der erfinderischen Pulvermischung, Granulate, Tabletten, Kapseln, Filmtabletten verlängert, ist es ratsam, das erfinderische Trockenmittel in einer gröberen Partikelgröße einzusetzen. Dadurch wird in Folge einer kleineren Partikeloberfläche während der Expositionszeit die Wasseraufnahme des hygroskopischen Trockenmittels verringert. Bewährt haben sich hier bei Trimagnesiumdicitrat und Calciumchlorid Partikelgrößen zwischen 0,2 und 2 mm mit einer bevorzugten mittleren Partikelgröße von ca. 0,8 mm. Ist der Wirkstoff dagegen besonders feuchtigkeitsempfindlich und es muss das durch den Einsatz von Hilfsstoffen unvermeidlich in die Mischung eingeschleppte Wasser rasch entfernt werden, empfiehlt sich der Einsatz von z.B. mikronisiertem Trimagnesiumdicitrat. Dieses Material hat eine bevorzugte mittlere Korngröße von 0,025 mm.

Bei der Herstellung von festen Formen mit feuchtempfindlichen Wirkstoffen entsprechend der Erfindung versucht man natürlich bevorzugt, getrocknete Hilfsstoffe bzw. Hilfsstoffe mit einem niedrigen Trocknungsverlust bzw. Hilfsstoffe mit sehr fest gebundenem Wasser einzusetzen. Die Herstellung von Pulvern, Granulaten, Kapseln, Tabletten erfolgt in der Regel ohne eine Feuchtgranulation. Dazu werden die Komponenten mit dem feuchtempfindlichen Wirkstoff gemischt und dann z.B. in dichte Sachets, Hartgelatinekapseln abgefüllt bzw. zu Tabletten verpresst. Häufig sind die Wirkstoffe und die notwendigen wasserarmen Hilfsstoffe nicht gut fließfähig, so dass der Fachmann eine Trockengranulation in der Weise durchführt, dass er die Mischung tablettiert oder kompaktiert und dann in der Weise siebt, dass ein gut fließfähiges trockenes Granulat entsteht. Grundsätzlich können alle bei der Herstellung von Granulaten, Kapseln oder Tabletten übliche Hilfsstoffe, sofern sie einen niedrigen Trocknungsverlust besitzen bzw. vorgetrocknet wurden, eingesetzt werden. Besonders geeignet sind vorgetrocknete mikrokristalline Cellulose, sprühgetrocknete, gut tablettierbare Laktose, Mannit, Xylit und Sprengmittel wie quervernetztes Povidon oder quervernetzte Natriumcarboxymethylcellulose. Als Fließreguliermittel ist z.B. Siliciumdioxid (mit dem Handelsnamen Syloid AL 1) besonders geeignet. Typische Schmiermittel für die Herstellung von Tabletten wie Magnesiumstearat und Stearinsäure, können problemlos verwendet werden, da sie nur geringe Mengen an Feuchtigkeit enthalten und selbst in der Regel nur in einer Menge von 0,5% eingesetzt werden.

Eine weitere Aufgabe für den Entwicklungsfachmann besteht darin, neben der Auswahl geeigneter Hilfsstoffe die Mengen an feuchtigkeitsempfindlichem Wirkstoff, für den Herstellprozess notwendige Hilfsstoffe und die bevorzugten Trocknungsmittel so zu wählen, dass sich Pulver und Granulate in Sachets oder Kapseln abfüllen lassen, ausreichend harte, mechanisch stabile Tabletten entstehen, die im Einzelfall bevorzugt mit einer alkoholischen Coatinglösung sogar in Filmtabletten überführt werden können. Selbstverständlich muss der Fachmann bei der qualitativen und quantitativen Auswahl der Hilfsstoffe und der bevorzugten Trockenmittel auch die Gesichtspunkte wie Zerfallszeit der Tabletten und Kapseln und die Freisetzungsgeschwindigkeit des Wirkstoffes aus diesen festen Formen im Auge behalten.

Die Menge an bevorzugtem Trocknungsmittel muss der Fachmann so wählen, dass

a) nach der Abfüllung sich eine entsprechend der Feuchtigkeitsempfindlichkeit des Wirkstoffes entsprechende Gleichgewichtsfeuchte in der festen Form einstellt, die sich aus Stabilitätsuntersuchungen bewährt hat
b) die während der Herstell- und Abfülloperationen unvermeidliche Feuchtigkeitsaufnahme kompensiert wird und die stabilitätsnotwendige Gleichgewichtsfeuchte sich in dem dichten Packmittel einstellt.

Glücklicherweise zeigen Calciumchlorid und insbesondere Trimagnesiumdicitrat günstiges Verarbeitungsverhalten, so dass z.B. bei der trockenen Granulierung selbst größter Mengen an Trockenmittel keine Schwierigkeiten entstehen. Da beide bevorzugten Hilfsstoffe auch wasserlöslich sind, treten somit auch keine Probleme bezüglich der Wirkstofffreisetzung auf. In Abhängigkeit von der Menge des zu verarbeitenden feuchtempfindlichen Wirkstoffes und insbesondere des Grades an seiner Feuchtigkeitsempfindlichkeit bestehen die festen Formen aus 10 bis 99,5, bevorzugt 15 bis 75 und ganz bevorzugt 20 bis 50% an den genannten Trockenmitteln.

Ein weiterer Aspekt der Erfindung ist die Verwendung von Trimagnesiumdicitrat zur Stabilisierung feuchtempfindlicher Wirkstoffe oder zur Erhöhung der Stabilität feuchtempfindlicher Wirkstoffe. Vorzugsweise wird die chemische Stabilität der Wirkstoffe erhöht. In einer besonderen Ausführungsform wird Trimagnesiumdicitrat verwendet um den Wirkstoff gegen Hydrolyse zu schützen.

Noch ein anderer Aspekt der Erfindung ist die Verwendung von Trimagnesiumdicitrat als Trockenmittel in festen Arzneiformen.

Schließlich betrifft die Erfindung ein Verfahren zur Erhöhung der Stabilität von feuchtempfindlichen Wirkstoffen, dadurch gekennzeichnet, dass man den Wirkstoff mit Trimagnesiumdicitrat mischt.

Die letztgenannten Aspekte der Erfindung können selbstverständlich mit den weiter oben beschriebenen Ausführungsformen anderer Aspekte kombiniert werden.

Die Erfindung wird durch die nachfolgenden Beispiele näher erläutert, ohne darauf eingeschränkt zu sein.

**Beispiel 1**

Trocknet man das bevorzugte Trocknungsmittel Trimagnesiumdicitrat mehrere Stunden bei 130°C, so erhält man ein Produkt, das zur Überraschung des Fachmannes eine Gleichgewichtsfeuchte mit einem kaum noch messbaren Wert von 0,6% bei 25°C aufweist.

Natriumsulfat, das einen Trocknungsverlust bei 130°C (Prüfzeit 30 min) von 0,06% aufweist, ergibt trotzdem bei der Bestimmung der Gleichgewichtsfeuchte einen Wert von 32,7%. Dieser Wert belegt, dass Natriumsulfat weder nennenswerte Menge Wasser adsorbieren kann geschweige diese adsorbierten Mengen fest bindet. Diese Substanz ist zur Stabilisierung von feuchtempfindlichen Wirkstoffen vollkommen ungeeignet.

**Tabelle 1**

|  | Trocknungsverlust bei 130°C/30 min | Gleichgewichtsfeuchte | Gewichtszunahme (%) bei Lagerung 15% rel. Feuchte/25°C |
|---|---|---|---|
| Mikrokristalline Cellulose | 0,5 | < 1,0 | 2,2 |
| Trimagnesium-dicitrat | 0,2 | < 1,0 | 8,6 |
| Calciumchlorid | 0,02 | < 1,0 | 45,9 |
| Sorbitol | 0,1 | 9,0 | 0,1 |
| Siliciumdioxid | 0,5 | < 1,0 | 6,4 |

Die Substanzen wurden 5 Stunden bei 130°C getrocknet (Sorbit: 70°C) und der Trocknungsverlust bestimmt. Anschließend wurde bei 25°C/15% rel. Feuchte die Gewichtszunahme der einzelnen Substanzen über 27 Tage bestimmt.

Die Tabelle belegt, dass Calciumchlorid selbst bei einer sehr geringen Feuchtigkeit von 15% größere Mengen an Wasser aufnehmen kann, dass aber zur Überraschung des Fachmannes das bevorzugte Trimagnesiumdicitrat mehr Wasser bindet als das allgemein bekannte Trockenmittel Siliciumdioxid (Silicagel).

**Beispiel 2**

In einem dicht schließenden Glasgefäß werden zwei Urgläschen mit 3,5 g Trimagnesiumdicitrat, getrocknet bei 130°C, bzw. mit 1,264 g Amoxicillin-Trihydrat (Wassergehalt 13.1%) nebeneinander gestellt und bei 25°C gelagert. Nach 14 Tagen beträgt das Gewicht des Amoxicillins nur noch 1.105g, das Gewicht des Trimagnesiumdicitrates jedoch 3,66g. Das Amoxicillin hat 12,6% Kristallwasser abgegeben, welches durch das Trockenmittel aufgenommen wurde. Das neue Trockenmittel ist so aktiv, dass es praktisch das gesamte Kristallwasser des Amoxicillins abgespalten hat (theoretischer Wert: 12,9%).

Wiederholt man diesen Versuch mit dem bekannten Trockenmittel Silicagel, so verliert das Antibiotikum nach 25 Tagen nur 0,1% an Gewicht. Dies bedeutet, dass das erfindungsgemäß getrocknete Trimagnesiumdicitrat ein außerordentlich potentes Trockenmittel darstellt.

Unter exakt gleichen Versuchsbedingungen verliert das Amoxicillin mit bei 130°C getrockneten Calciumchlorid nach 26 Tagen/25°C nur 9,0%.

**Beispiel 3**

Es wurden zwei trockene Mischungen hergestellt mit nachfolgender Zusammensetzung:

**Tabelle 2**

|  | a) | b) |
|---|---|---|
| Acetylsalicylsäure | 500mg | 500mg |
| Sorbit | 950mg | 950mg |
| Zitronensäure | 59mg | 59mg |
| Magnesiumoxid | 25mg | 25mg |
| Aspartam | 10mg | 10mg |
| Zitronenaroma | 25mg | 25mg |
| Trimagnesiumdicitrat | --- | 125mg |

Die Komponenten wurden gemischt, in Aluminium-kaschierte Beutel gefüllt und drei Monate bei 40°C gelagert.

Im Falle a) entstand eine durch Wasser ausgelöste Zersetzung der Acetylsalicylsäure unter Bildung von 3,2% Salicylsäure und Eisessig. Das Muster war ungenießbar.

Das Muster b) zeigte eine Zersetzung von 0,6% und praktisch keinen Geruch von Eisessig. Das Muster war geschmacklich noch in Ordnung.

Die Gleichgewichtsfeuchte des Musters a) war 34%(25°C), des Musters b) infolge des Zusatzes von Trimagnesiumdicitrat 8,4%. Die erfindungsgemäße niedrige Gleichgewichtsfeuchte stabilisiert durch seine Wasser-Bindung den Wirkstoff deutlich.

**Beispiel 4**

Trimagnesiumdicitrat, Calciumchloridx2H$_2$O werden bei 130°C mehrere Stunden getrocknet. Der Trocknungsverlust, gemessen bei 150°C/30 min beträgt:
Trimagnesiumdicitrat: 1,6%; Gleichgewichtsfeuchte < 0,5%
Calciumchlorid: 3,4%; Gleichgewichtsfeuchte < 0,5%

| | |
|---|---|
| Clavulansäure | 3,0 kg |
| Trimagnesiumdicitrat (mittlere Teilchengröße 0,25 mm) | 4,0 kg |
| Mikrokristalline Cellulose | 1,5 kg |
| Mannit | 1,0 kg |
| Siliciumdioxid (Syloid AL 1) | 0,2 kg |
| Povidone K25 | 0,2 kg |
| Magnesiumstearat | 0,1 kg |

Die Komponenten wurden in einem Raum bei 22°C, 19% rel. Feuchte gesiebt und anschließend in einem Walzenkompaktor zu einer Schülpe verdichtet. Die Schülpe wurde über 2,5 mm und 1,0 mm trockengranuliert. Das Granulat wurde vor der Verkapselung in einem dicht schließenden Behälter aufbewahrt.
Der Trocknungsverlust des Granulates, gemessen bei 105°C/30 min betrug 2,4%, die Gleichgewichtsfeuchte 4,6% (25°C). Unter exakt gleichen Bedingungen wurde der Versuch mit 3,0 kg getrocknetem Calciumchlorid wie oben beschrieben wiederholt. Das Calciumchlorid ließ sich ähnlich gut zu einem Granulat verarbeiten.

Trocknungsverlust: 1,7% (105/30 min)
Gleichgewichtsfeuchte: 5,4% (25°C)

Beide Granulate wurden bei 21°C/22% rel. Feuchte in ungetrocknete HPMC-Kapseln (Füllgewicht 417 mg pro Kapsel-Größe 1) und unter den gleichen Raumbedingungen in Glas-Fläschchen abgefüllt (20 Stück) und mit dicht schließenden PE-Stopfen verschlossen. Einige Stopfen enthielten 2,0 g Molekularsieb.
Die Muster wurden einem Stresstest über 6 Monate bei 40°C/75% rel. Feuchte unterzogen.

**Tabelle 3**

| Testparameter | Start | 3 Monate | 6 Monate | 6 Monate* |
|---|---|---|---|---|
| Aussehen Kapselinhalt | weiß | weiß | fast weiß | fast weiß |
| Zerfallszeit Kapsel in Wasser, 37°C | 8,5 min | 9,2 min | 9,1 min | 8,8 min |
| Trocknungsverlust Kapselinhalt | 2,8% | 2,6% | 2,9% | 2,5% |
| Gleichgewichtsfeuchte Kapselinhalt | 5,2% | 4,8% | 5,5% | 4,2% |
| Trocknungsverlust HPMC-Kapsel | 2,7% | 2,3% | 2,2% | 2,1% |
| Trocknungsverlust Trockenmittel/Stopfen | 1,2% | - | - | 1,6% |
| Gehalt Clavulansäure | 101,4% | 99,8% | 96,1% | 96,7% |
| * Muster mit Trockenstopfen Alle Trocknungsverluste gemessen bei 105°C/30 min, Trocknungsverlust Trockenmittel/Stopfen bei 200°C/30 min | | | | |

Ergebnisse (Clavulansäure-Kapseln mit Calciumchlorid):

**Tabelle 4**

| Testparameter | Start | 3 Monate | 6 Monate | 6 Monate* |
|---|---|---|---|---|
| Aussehen Kapselinhalt | weiß | fast weiß | gelb | weiß/ gelb |
| Zerfallszeit Kapsel in Wasser, 37°C | 7,9 min | 8,2 min | 8,6 min | 8,4 min |
| Trocknungsverlust Kapselinhalt | 2,1% | 2,3% | 2,2% | 1,7% |
| Gleichgewichtsfeuchte Kapselinhalt | 5,9% | 6,9% | 7,2% | 5,2% |
| Trocknungsverlust HPMC-Kapsel | 3,1% | 3,2% | 2,9% | 2,5% |
| Trocknungsverlust Trockenmittel/Stopfen | 1,2% | - | - | 6,4% |
| Gehalt Clavulansäure | 100,7% | 98,7% | 93,1% | 94,6% |
| * Muster mit Trockenstopfen<br>Alle Trocknungsverluste bei 105°C / 30 min<br>Trocknungsverlust Trockenmittel/Stopfen bei 200°C /30 min | | | | |

Obwohl die Trocknungsverluste des Kapselinhaltes bei beiden Versuchsbeispielen infolge Expositionszeit während der Kapselabfüllung und des Wassergehaltes der ungetrockneten Kapsel ansteigt, verändert sich praktisch die Gleichgewichtsfeuchte des Kapselinhaltes bei beiden Versuchsbeispielen nicht. Dies beweist, dass das während der Abfüllung aufgenommene und aus der Kapsel entzogene Wasser von dem Trockenmittel aufgenommen wird ohne dass die Gleichgewichtsfeuchte, die für die Stabilität der feuchtempfindlichen Clavulansäure entscheidend ist, sich verändert. Auch in Folge des dichten Packmittels verändern sich während der Stresslagerung über 3 und 6 Monate die Trocknungsverluste des Kapselinhaltes und die dazu gehörigen Gleichgewichtsfeuchten kaum. Dies ist eine wesentliche Vorraussetzung für die Stabilisierung des Wirkstoffes. Hochinteressant sind die Ergebnisse zum Trocknungsverlust des Trockenmittels in den Stopfen. Obwohl dem Fachmann bekannt ist, dass Molekularsieb ein extrem scharfes Trockenmittel ist, hat es zur Überraschung des Fachmannes im Falle des Trockenmittels Timagnesiumdicitrat kaum Wasser aus den Kapseln mit Inhalt herausgezogen und der Trocknungsverlust ist nur von 1,2 auf 1,6% gestiegen. Dies ist ein klarer Beweis für die starke Bindung des Wassers an Trimagnesiumdicitrat. Wiederum zur Überraschung des Fachmannes ist die Wasseraufnahme des Molekularsiebs im Falle der Clavulankapseln mit dem Trockenmittel Calciumchlorid deutlich von 1,2% auf 6,4% angestiegen. Obwohl Calciumchlorid als starkes Trockenmittel gilt, hat das Molekularsieb über 5% Wasser aus den Kapseln aufgenommen. Dies wiederum beweist, dass Trimagnesiumdicitrat zur Überraschung des Fachmannes zwar weniger Wasser binden kann als Calciumchlorid, dass aber die Stärke der Wasserbindung deutlich höher ist. Dies macht Trimagnesiumdicitrat zum bevorzugten Trockenmittel für hochwasserempfindliche Wirkstoffe, da das neue Trockenmittel in der Lage ist, bis zu den Bedingungen von ca. 40°C sozusagen das Wasser irreversibel zu binden und den empfindlichen Wirkstoff weitgehend auszutrocknen, indem es in der festen Form Gleichgewichtsfeuchten um 5% bis zu Temperaturen von 40°C garantieren kann.

Stellt man das Clavulansäure-Granulat ohne eines der beiden Trockenmittel her, so hat es nach der Herstellung eine Gleichgewichtsfeuchte von 28,9%, welches eine für Kapselgranulate sehr niedrige Gleichgewichtsfeuchte bedeutet. Der Wirkstoff in dieser Mischung verliert innerhalb einer Woche, gelagert bei 40°C in einer dichten Flasche, mehr als 25% Gehalt und verfärbt sich nach hellbraun. Normale Kapselmischungen haben eine Gleichgewichtsfeuchte von 35 bis 55%. Es handelt sich also in der Tat bei Clavulansäure um einen äußerst feuchtigkeitsempfindlichen Wirkstoff, der selbst in Gegenwart von geringen Mengen Wasser rasch an Aktivität verliert.

**Patentansprüche**

1. Feste Arzneiform enthaltend wenigstens einen Wirkstoff und wenigstens ein pharmazeutisch verträgliches, wasserlösliches Trockenmittel, das ausgewählt ist aus der Gruppe bestehend aus Trimagnesiumdicitrat, Calciumchlorid und Kombinationen davon, **dadurch gekennzeichnet, dass** die feste Arzneiform einen Trocknungsverlust, gemessen bei 120°C/30 min, von höchstens 6% und eine relative Gleichgewichtsfeuchte, gemessen bei 25°C, von 25% oder weniger aufweist.

2. Feste Arzneiform nach Anspruch 1, **dadurch gekennzeichnet, dass** die feste Arzneiform eine relative Gleichgewichtsfeuchte von 10% oder weniger aufweist.

3. Feste Arzneiform nach Anspruch 1 oder 2, **dadurch gekennzeichnet, dass** die Konzentration des Trockenmittels in der festen Arzneiform 15 bis 75 Gew.-% ist.

4. Feste Arzneiform nach einem der Ansprüche 1 bis 3, **dadurch gekennzeichnet, dass** das Trockenmittel Trimagnesiumdicitrat ist.

5. Feste Arzneiform nach einem der Ansprüche 1 bis 3, **dadurch gekennzeichnet, dass** das Trockenmittel Calciumchlorid ist.

6. Feste Arzneiform nach einem der vorhergehenden Ansprüche, **dadurch gekennzeichnet, dass** die Konzentration des Wirkstoffs in der festen Arzneiform 5 bis 50 Gew.-% ist.

7. Feste Arzneiform nach einem der vorhergehenden Ansprüche, **dadurch gekennzeichnet, dass** der Wirkstoff gegen Feuchtigkeit empfindlich ist.

8. Feste Arzneiform nach einem der vorhergehenden Ansprüche, **dadurch gekennzeichnet, dass** die feste Arzneiform eine Tablette, Kapsel, ein Granulat oder ein Pulver ist.

9. Feste Arzneiform enthaltend wenigstens einen feuchtempfindlichen Wirkstoff, 5 bis 95 Gew.-% Trimagnesiumdicitrat und gegebenenfalls weitere Hilfsstoffe.

10. Feste Arzneiform nach Anspruch 9, **dadurch gekennzeichnet, dass** sie 30 bis 40% feuchtempfindlichen Wirkstoff und 30 bis 40 % Trimagnesiumdicitrat enthält.

11. Feste Arzneiform nach Anspruch 9 oder 10, **dadurch gekennzeichnet, dass** sie weiterhin 10 bis 20 Gew.-% mikrokristalline Cellulose, 0,2 bis 2% Schmiermittel und 1 bis 5% Gleitmittel enthält.

12. Feste Arzneiform nach einem der Ansprüche 9 bis 11, **dadurch gekennzeichnet, dass** sie einen Trocknungsverlust von 0,5 bis 3%, gemessen bei 120°C/30 min, und eine relative Gleichgewichtsfeuchte, gemessen bei 25°C, von weniger als 10% aufweist.

13. Feste Arzneiform nach einem der vorhergehenden Ansprüche, **dadurch gekennzeichnet, dass** sie in einem dichten Packmittel verpackt ist.

14. Verfahren zur Herstellung einer festen Arzneiform nach einem der vorhergehenden Ansprüche, umfassend die folgenden Schritte:

   a) Mischen wenigstens eines Wirkstoffs mit einem Trockenmittel, das ausgewählt ist aus der Gruppe bestehend aus Trimagnesiumdicitrat, Calciumchlorid und Kombinationen davon,
   b) gegebenenfalls Trockengranulierung der in Schritt a) erhaltenen Zusammensetzung, um ein Granulat zu erhalten,
   c) komprimieren oder verkapseln der in Schritt a) erhaltenen Zusammensetzung oder des in Schritt b) erhaltenen Granulats, um die feste Arzneiform zu erhalten, und
   d) gegebenenfalls Verpacken der festen Arzneiform in ein dichtes Packmittel,

   wobei das Verhältnis der Masse an Trockenmittel zu der Masse an Wirkstoff in Schritt a) so gewählt wird, und das Verfahren unter solchen Bedingungen durchgeführt wird, dass die in Schritt c) erhaltene feste Arzneiform einen Trocknungsverlust, gemessen bei 120°C/30 min, von höchstens 6% und eine relative Gleichgewichtsfeuchte, gemessen bei 25°C, von 25% oder weniger aufweist.

15. Verfahren nach Anspruch 14, **dadurch gekennzeichnet, dass** das Trockenmittel im wesentlichen wasserfreies Trimagnesiumdicitrat ist.

16. Verfahren nach Anspruch 14 oder 15, **dadurch gekennzeichnet, dass** das Verhältnis der Masse des Trockenmittels zu der Masse des Wirkstoffs in Schritt a) 0,75 bis 1,5 ist.

17. Verfahren zur Erhöhung der chemischen Stabilität eines feuchtempfindlichen Wirkstoffs, **dadurch gekennzeichnet, dass** man den Wirkstoff mit Trimagnesiumdicitrat mischt.

**18.** Verwendung von Trimagnesiumdicitrat zur Stabilisierung feuchtempfindlicher Wirkstoffe.

**19.** Verwendung von Trimagnesiumdicitrat zum Schutz feuchtempfindlicher Wirkstoffe gegen Hydrolyse.

**20.** Verwendung von Trimagnesiumdicitrat als Trockenmittel in festen Arzneiformen.

**Europäisches Patentamt**

**EUROPÄISCHER RECHERCHENBERICHT**

Nummer der Anmeldung

EP 06 02 0008

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | WO 97/17960 A1 (SMITHKLINE BEECHAM S.P.A., ITALY; FONIO, TEODORO; PESATORI, MARCO; OLD) 22. Mai 1997 (1997-05-22) | 1-8 | INV. A61K9/20 A61K9/28 A61K9/48 A61K9/00 |
| X | Beispiel, Seite 5; Seite 3, Zeilen 23-25; Ansprüche ----- | 1-20 | |
| Y | DE 199 62 251 A1 (HERMES FABRIK PHARM. PRAEPARATE FRANZ GRADINGER G.M.B.H. & CO., GERMAN) 6. September 2001 (2001-09-06) Beispiel 1, Column 1, Zeile 46 ff, Ansprüche, inbes. Ansprüche 6-8 ----- | 1-20 | |
| Y | WO 93/13760 A (GERGELY GERHARD [AT]) 22. Juli 1993 (1993-07-22) Beispiel 4, Seite 9 ----- | 1-20 | |
| Y | US 4 537 887 A (ROOKE DAVID J [GB] ET AL) 27. August 1985 (1985-08-27) Column 2, Ansprüche ----- | 1-20 | |
| Y | "Desiccant prodn. - by heating deliquescent mixt. of calcium-, magnesium- and lithium chloride(s) sodium hydroxide, aq. binder and/ inorganic cpd" DERWENT, 10. Januar 1983 (1983-01-10), XP002204805 * Zusammenfassung * ----- | 1-20 | RECHERCHIERTE SACHGEBIETE (IPC) A61K |
| A | UMAGOE O ET AL: "MANUFACTURE OF MAGNESIUM CHLORIDE ANHYDRIDE FOR DESICCANT" CHEMICAL ABSTRACTS + INDEXES, AMERICAN CHEMICAL SOCIETY. COLUMBUS, US, Bd. 109, Nr. 18, 1. Oktober 1988 (1988-10-01), Seite 177, XP000015879 ISSN: 0009-2258 * Zusammenfassung * ----- -/-- | 1-20 | |

Der vorliegende Recherchenbericht wurde für alle Patentansprüche erstellt

| Recherchenort | Abschlußdatum der Recherche | Prüfer |
|---|---|---|
| München | 13. September 2007 | KRONESTER-FREI, A |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

.......................................................

& : Mitglied der gleichen Patentfamilie, übereinstimmendes Dokument

EPO FORM 1503 03.82 (P04C03)

**Europäisches Patentamt**

**EUROPÄISCHER RECHERCHENBERICHT**

Nummer der Anmeldung

EP 06 02 0008

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | WO 2004/004754 A1 (BOEHRINGER INGELHEIM PHARMA G.M.B.H. & CO. K.-G., GERMANY) 15. Januar 2004 (2004-01-15) * das ganze Dokument * ----- | 1-20 | |

RECHERCHIERTE SACHGEBIETE (IPC)

Der vorliegende Recherchenbericht wurde für alle Patentansprüche erstellt

| Recherchenort | Abschlußdatum der Recherche | Prüfer |
|---|---|---|
| München | 13. September 2007 | KRONESTER-FREI, A |

EPO FORM 1503 03.82 (P04C03)

EP 1 902 708 A1

## ANHANG ZUM EUROPÄISCHEN RECHERCHENBERICHT
### ÜBER DIE EUROPÄISCHE PATENTANMELDUNG NR.

EP 06 02 0008

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

13-09-2007

| Im Recherchenbericht angeführtes Patentdokument | | | Datum der Veröffentlichung | Mitglied(er) der Patentfamilie | | | Datum der Veröffentlichung |
|---|---|---|---|---|---|---|---|
| WO 9717960 | A1 | | 22-05-1997 | IT | MI952354 | A1 | 16-05-1997 |
| DE 19962251 | A1 | | 06-09-2001 | KEINE | | | |
| WO 9313760 | A | | 22-07-1993 | AT | 128028 | T | 15-10-1995 |
| | | | | AU | 3349293 | A | 03-08-1993 |
| | | | | CA | 2127352 | A1 | 22-07-1993 |
| | | | | CZ | 9401605 | A3 | 15-12-1994 |
| | | | | DE | 59300632 | D1 | 26-10-1995 |
| | | | | ES | 2068800 | T1 | 01-05-1995 |
| | | | | FI | 943315 | A | 12-07-1994 |
| | | | | GR | 95300005 | T1 | 28-02-1995 |
| | | | | GR | 3017806 | T3 | 31-01-1996 |
| | | | | JP | 7506336 | T | 13-07-1995 |
| | | | | MX | 9300110 | A1 | 29-07-1994 |
| | | | | PL | 173676 | B1 | 30-04-1998 |
| | | | | US | 5587179 | A | 24-12-1996 |
| | | | | ZA | 9300215 | A | 19-08-1993 |
| US 4537887 | A | | 27-08-1985 | AU | 549321 | B2 | 23-01-1986 |
| | | | | AU | 7568481 | A | 08-04-1982 |
| | | | | CA | 1187796 | A1 | 28-05-1985 |
| | | | | DE | 3172028 | D1 | 03-10-1985 |
| | | | | EP | 0049061 | A1 | 07-04-1982 |
| | | | | ES | 8307095 | A1 | 16-10-1983 |
| | | | | GB | 2084016 | A | 07-04-1982 |
| | | | | GR | 75049 | A1 | 12-07-1984 |
| | | | | HK | 17386 | A | 21-03-1986 |
| | | | | IE | 51597 | B1 | 21-01-1987 |
| | | | | JP | 1055244 | B | 22-11-1989 |
| | | | | JP | 1568566 | C | 10-07-1990 |
| | | | | JP | 57091921 | A | 08-06-1982 |
| | | | | MY | 35386 | A | 31-12-1986 |
| | | | | NZ | 198241 | A | 16-12-1983 |
| | | | | PT | 73686 | A | 01-10-1981 |
| | | | | ZA | 8106272 | A | 29-09-1982 |
| WO 2004004754 | A1 | | 15-01-2004 | AU | 2003281249 | A1 | 23-01-2004 |
| | | | | CA | 2491712 | A1 | 15-01-2004 |
| | | | | DE | 10230752 | A1 | 22-01-2004 |
| | | | | EP | 1521590 | A1 | 13-04-2005 |
| | | | | JP | 2005532377 | T | 27-10-2005 |

Für nähere Einzelheiten zu diesem Anhang : siehe Amtsblatt des Europäischen Patentamts, Nr.12/82

EPO FORM P0461

16

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- **W. A. RITSCHEL ; A. BAUER-BRANDL.** Die Tablette. Cantor Verlag Aulendorf, 2002, 286